Europäisches Patentamt

European Patent Office

Office européen des brevets

(11) Veröffentlichungsnummer: **0 264 610**

**A1**

(12) **EUROPÄISCHE PATENTANMELDUNG**

(21) Anmeldenummer: **87113314.6**

(22) Anmeldetag: **11.09.87**

(51) Int. Cl.⁴: **A61F 13/20**

(30) Priorität: **11.10.86 DE 3634682**

(43) Veröffentlichungstag der Anmeldung:
**27.04.88 Patentblatt 88/17**

(84) Benannte Vertragsstaaten:
**AT BE CH ES FR GB GR IT LI LU NL**

(71) Anmelder: **Vereinigte Papierwerke AG**
**Schoppershofstrasse 80**
**D-8500 Nürnberg(DE)**

(72) Erfinder: **Rink, Norbert, Dr.**
**Wodanstrasse 17**
**D-8508 Wendelstein(DE)**
Erfinder: **Golovatai-Schmidt, Eduard**
**Paradiesstrasse 9**
**D-8500 Nuernberg(DE)**
Erfinder: **Stary, Christof**
**Am Steinbruch 11**
**D-8501 Eckental(DE)**

(74) Vertreter: **Pohl, Hans Ludwig**
**Hefnersplatz 3**
**D-8500 Nürnberg 11(DE)**

(54) **Gepresster stäbchenförmiger Tampon für die Frauenhygiene.**

(57) Es wird ein gepreßter stäbchenförmiger Tampon fir die Frauenhygiene beschrieben, bei dem ein mit einem Rückholfaden verbundener und spiralförmig zum Tamponkern aufgewickelter Wattezopf allseitig mit einem Vliesstoffmantel umgeben und zusammen mit diesem radial verpreßt ist. Die Länge des Vliesstoffstreifens ist so gewählt, daß der Streifen den Wattewickel unter Bildung einer Überlappungszone vollständig mantelförmig umgibt. Die Breite des Vliesstoffstreifens ist so gewählt, daß der Streifen den Wickel oben und unten überragt. Der Vliesstoffmantel (4) ist am Stirnende (6) und am Fußende (7) jeweils durch eine Siegelnaht (8;9) verschlossen und die beiden Siegelnähte sind beim fertigen Tampon an die Stirn-bezw. Fußfläche angepreßt.

Fig. 2

## Gepreßter stäbchenförmiger Tampon für die Frauenhygiene

Die Erfindung betrifft einen gepreßten stäbchenförmigen Tampon für die Frauenhygiene, bei dem ein mit einem Rückholfaden verbundener und spiralförmig zum Tamponkern aufgewickelter Wattezopf allseitig mit einem Vliesstoffmantel umgeben und zusammen mit diesem radial verpreßt ist, wobei die Länge des Vliesstoffstreifens so gewählt ist, daß der Streifen den Wattewickel unter Bildung einer Oberlappungszone vollständig mantelförmig umgibt und wobei die Breite des Vliesstoffstreifens so gewählt ist, daß der Streifen den Wickel oben und unten überragt. Derartige Tampons sind beispielsweise aus der DE-PS 28 54 720 bekannt. Der Vliesstoffmantel hat den Zweck, den Tamponkern flusendicht zu umhüllen, damit beim Gebrauch und nach dem Entfernen keine Flusen in der Körperhöhle des Benutzers zurückbleiben können. Einen darüber hinausgehenden Zweck hat der dort beschriebene Vliesstoffmantel nicht.

Bei Tampons mit spiralförmig aufgewickeltem Wattekern besteht ein wichtiges Problem darin, das Entspiralisieren des Wattekerns beim Entfernen des gebrauchten Tampons zu verhindern. Um dieses Problem zu lösen ist beim Tampon gemäß DE-PS 28 54 720 der Rückholfaden auf komplizierte Weise um die einzelnen Windungen des aufgewickelten Tamponkerns geführt. Die Anordnung des Rückholfadens in dieser Weise ist kompliziert und erfordert Maschinen, die nur mit verhältnismäßig geringer Taktzahl arbeiten können. Die Herstellung wird noch dadurch erschwert, daß, wie gesagt, der Vliesstoffmantel am oberen und unteren Ende in das Innere des Tamponkerns eingesenkt werden muß, was ebenfalls maschinentechnisch schwierig zu bewirken ist.

Der Erfindung liegt die Aufgabe zugrunde, die vorbekannten Tampons dahingehend weiterzuentwickeln, daß keine besondere und komplizierte Führung des Rückholfadens im Innern des Tamponwickels erforderlich ist, und daß auch das Einstülpen des Tamponmantels an der Stirnfläche sowie in der Fußfläche des Tamponrohlings in das Innere des Tamponwikkels entfällt.

Zur Lösung dieser Aufgabe wird von dem Gedanken ausgegangen, dem Vliesstoffmantel noch eine weitere Funktion zuzuweisen, die in der Verhinderung des Entspiralisierens besteht. Die Lösung ist dadurch gekennzeichnet, daß der Vliesstoffmantel am Stirnende und am Fußende jeweils durch eine Siegelnaht verschlossen ist und die beiden Siegelnähte beim fertigen Tampon an die Stirn-bzw. Fußfläche angepreßt sind. Durch den oberen und unteren Verschluß des Tamponmantels wird die Vliesstoffhülle zu einem Gebilde, welches den Tamponkern käfigartig umgibt und ihn auch nach Gebrauch, also im gesättigten und ausgedehnten Zustand, spiralförmig zusammenhält. Wird über den Rückholfaden eine Zugkraft auf die innere Lage des spiralförmig gewickelten Tamponkerns ausgeübt, so kann der Kern dennoch nicht entspiralisieren, da der Mantel, der den Kern käfigartig umgibt, ihn am Auseinandergleiten der einzelnen Kernwicklungen hindert. Neben diesem erheblichen Gebrauchsvorteil wird zugleich noch der Vorteil erzielt, daß der Rückholfaden, wie an sich vorbekannt, den Wattezopf nur einfach umschlingen muß, und daß schnellaufende Maschinen zur Herstellung des Tamponrohlings verwendet werden können, da das Einstülpen des Mantels entfällt.

Der Gegenstand der Erfindung wird im folgenden anhand der beigefügten Zeichnung näher erläutert. Es stellen dar:

Fig. 1 eine vereinfachte perspektivische Darstellung eines Tamponrohlings mit umgelegtem, noch unverschlossenen Vliesstoffmantel;

Fig. 2 eine vereinfachte perspektivische Darstellung mit verschlossenem Vliesstoffmantel.

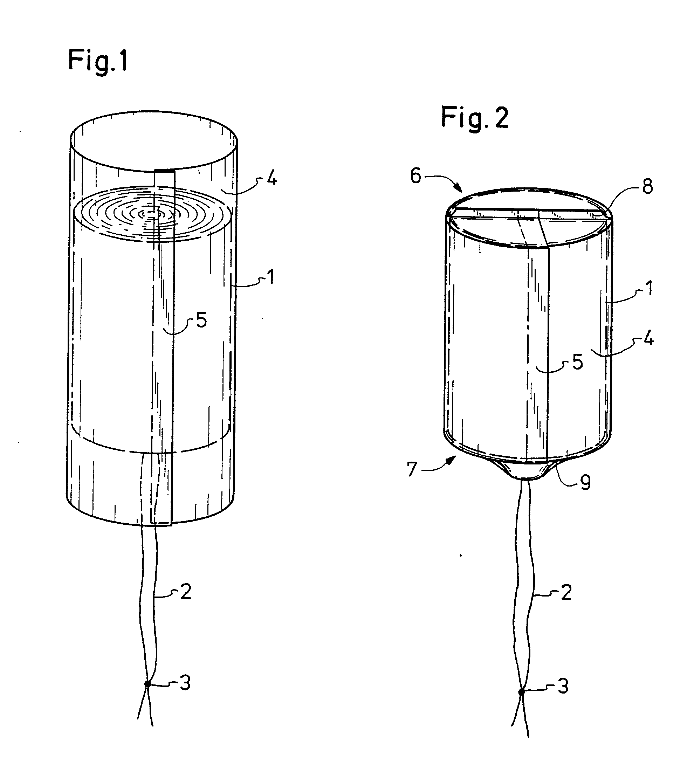

In Fig. 1 ist bei 1 ein spiralförmig aufgewickelter Wattezopf dargestellt. Dieser Zopf, der den Saügkern des fertigen Tampons bilden wird, ist in an sich bekannter Weise mit einem Rückholfaden 2 ausgestattet, dessen freie Enden bei 3 miteinander verknotet sind.

Der aufgewickelte Wattezopf ist mit einem Vliesstoffmantel 4 umgeben, der aus einem Vliesstoffstreifen gebildet worden ist. Die Länge des Vliesstoffstreifens ist dabei so gewählt, daß der Streifen den Wattewickel unter Bildung einer Oberlappungszone 5 vollständig mantelförmig umgibt. Die Breite des Vliesstoffstreifens ist so gewählt, daß der Streifen den Wickel oben und unten überragt.

Zum Verschluß des Vliesstoffmantels 4 wird dieser am Stirnende 6 sowie am Fußende 7 jeweils durch eine Siegelnaht 8; 9 verschlossen. Die Siegelnaht ist am unteren Ende des Tampons so geführt, daß sie den Rückholfaden frei passieren läßt. Auf diese Weise ist der Rückholfaden in Richtung seiner Längsachse verschiebbar, was wünschenswert sein kann, wenn die Maßnahme ergriffen werden soll, den Rückholfaden vor Gebrauch des Tampons im Innern des Wattezopfwickels oder des Vliesstoffmantels unterzubringen. Die Zeichnung, insbesondere Fig. 2, läßt erkennen, daß der Vliesstoffmantel 4 nach seinem Verschließen den Wattezopf wie ein Käfig umgibt. Nach Gebrauch hat sich der Tampon mit Flüssigkeit vollgesaugt und er hat wieder in etwa die Form angenommen, die in Fig. 2 für den Tamponrohling dargestellt ist. Wird in diesem Zustand eine Zugkraft über den Rückholfaden 2 auf den Wattezopf 1 ausgeübt, so kann sich dieser dennoch nicht entspiralisieren, da er, wie Fig. 2 erkennen läßt, durch den allseits zusammenhaltenden Vliesstoffmantel 4 am Auseinandergehen gehindert wird.

Der in Fig. 2 dargestellte Tamponrohling wird in an sich bekannter Weise durch radiales und gegebenenfalls leichtes längsaxiales Pressen zum fertigen Tampon weiterverarbeitet. Bei der Ausführung dieser Preßvorgänge ergibt es sich von selbst, daß die beiden Siegelnähte 8 und 9 an die Stirnfläche 6 bzw. die Fußfläche 7 angelegt werden, so daß die Nähte beim fertigen Tampon überhaupt nicht in Erscheinung treten und auch bei Gebrauch nicht stören.

Bezugzeichenliste

1 = Wattezopf
2 = Rückholfaden
3 = Knoten
4 = Vliesstoffmantel
5 = Überlappungszone
6 = Stirnende
7 = Fußende
8;9 = Siegelnaht

**Ansprüche**

Gepreßter stäbchenförmiger Tampon für die Frauenhygiene, bei dem ein mit einem Rückholfaden verbundener und spiralförmig zum Tamponkern aufgewickelter Wattezopf allseitig mit einem Vliesstoffmantel umgeben und zusammen mit diesem radial verpreßt ist,
wobei die Länge des Vliesstoffstreifens so gewählt ist, daß der Streifen den Wattewickel unter Bildung einer Oberlappungszone vollständig mantelförmig umgibt und wobei die Breite des Vliesstoffstreifens so gewählt ist, daß der Streifen den Wickel oben und unten überragt, dadurch gekennzeichnet, daß der Vliesstoffmantel (4) am Stirnende (6) und am Fußende (7) jeweils durch eine Siegelnaht (8, 9) verschlossen ist und die beiden Siegelnähte beim fertigen Tampon an die Stirn-bzw. Fußfläche angepreßt sind.

Fig. 1

Fig. 2

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| X | DE-U-8 535 520 (VER. PAPIERWERKE) * Insgesamt * --- | 1 | A 61 F 13/20 |
| A | FR-A-2 395 746 (KIMBERLY CLARK) --- | | |
| A | US-A-2 844 150 (DRAGHI) --- | | |
| D,A | GB-A-2 010 680 (MÖLNLYCKE) ----- | | |

RECHERCHIERTE
SACHGEBIETE (Int. Cl.4)

A 61 F

Der vorliegende Recherchenbericht wurde für alle Patentansprüche erstellt

| Recherchenort | Abschlußdatum der Recherche | Prüfer |
|---|---|---|
| DEN HAAG | 06-01-1988 | STEENBAKKER J. |